# EUROPEAN PATENT SPECIFICATION

(11) **EP 0 821 067 B1**
(45) Date of publication and mention of the grant of the patent: **31.03.2004**
(21) Application number: 97900606.1
(22) Date of filing: 13.01.1997
(51) Int. Cl.: C02F 3/28, C12R 1/225, A23C 21/00, C12P 7/56

(54) **PROCESS FOR TREATING RESIDUAL SERUM IN THE CHEESE FABRICATION, ELIMINATING ITS CONTAMINATING POWER**
VERFAHREN ZUR BEHANDLUNG VON RESTSERUM IN DER KÄSEHERSTELLUNG, UND ENTFERNUNG SEINES VERUNREINIGUNGSPOTENTIALS
PROCEDE DE TRAITEMENT DES SERUMS RESIDUELS LORS DE LA FABRICATION DE FROMAGE, PERMETTANT D'ELIMINER LEUR POUVOIR CONTAMINANT

(30) Priority: 08.02.1996 ES 9600301
(43) Date of publication of application: 28.01.1998
(73) Proprietor: Fuertes Gonzalez, Juan, 28003 Madrid (ES); Moya Mora, Angela M, 13004 Ciudad Real (ES)
(72) Inventor: Fuertes Gonzalez, Juan, 28003 Madrid (ES); Moya Mora, Angela M, 13004 Ciudad Real (ES)
(74) Representative: Ziebig, Marlene, Dr. Dipl.-Chem.
(86) International application number: PCT/ES1997/000006
(87) International publication number: WO 1997/029204

(56) References cited:
- EP-A- 0 147 517
- EP-A- 0 265 409
- EP-A- 0 517 242
- US-A- 4 676 987
- US-A- 4 699 793

## Description

This patent is related to a procedure for processing the whey which is produced as a by-product in the cheese-manufacturing industries, this being a by-product which constitutes a problem due to the contaminating features of its content, given that it is comprised of solid substances, particularly lactose, a readily-fermentable sugar, as well as a large number of microorganisms which might give rise to cases of undesirable fermentation.

These circumstances give rise to a serious problem as regards the effluents and disposal of the whey produced in the cheese-making industries, given that the laws currently in force are aimed at guaranteeing the conservation of the environment under the most favorable circumstances for the natural surroundings.

Apart from the above, in addition to affording the possibility of eliminating the undesirable contaminating effects of the aforesaid by-product, the implementation of this procedure also provides the considerable advantage of obtaining such an industrially valuable product as L-lactic acid as a final resulting by-product, whereas the production of L-lactic acid is not part of the invention.

It has been known for a long time that L-lactic acid is an essential additive in the food-preserving industry which is obtained by many known methods, some of which have been created specifically for the production thereof.

Starting in 1931, a lactobacillus was employed to produce L-lactic acid, that is, Lactobacillus Bulgaricus, to carry out a suitable fermentation at a controllable pH. The lactic acid contained in the fermentation medium was later removed using sulfuric acid, giving rise to an acid suitable for use in the food industry.

The aforesaid method entailed major drawbacks, given that the full use of the sugar employed was limited to 80%, and the separation and purification methods gave rise to greater degrees of contamination than those they were aimed at correcting.

Therefore, procedures were subsequently further developed aimed at preventing the aforementioned drawbacks. One of these procedures is the object of French Patent No. 83 18631, which claims a continuous fermentation system entailing different stages in which organic acids and sugars are finally obtained in the end, but which turns out to be a too highly involved industrial process.

A later procedure was described in U.S. Patent No. 4,698,303, but which also entailed a major drawback on it being necessary to eliminate the proteins of relatively high molecular weights in order to obtain the lactic acid, additionally requiring that the whey be centrifuged and subsequently ultrafiltered, which gave rise to specific problems regarding the strength of the membranes employed, entailing a major reduction in efficiency and the need of employing removal techniques using different solvents, the recovery of which would involve added complications and cases of contamination.

Although the two aforementioned patents afford the possibility of obtaining a lactic acid of a purity sufficing for its use in the food industry and for the manufacture of pharmaceutical products, it can also be used in industrial processes less stringent as regards the quality of the lactic acid obtained, some of which may include the manufacture of mordants for tanning, the treatment of other surfaces and the processing of chemical products, as the most significant, unrestricted examples.

From US-A 4,699,793 and EP-A 0 147 517 are known procedures for the manufacturing of products from the whey which are used in the food industry.

US-A 4,699,793 describes a method of producing a seasoning from cheese whey. In a first step multiplication factors (yeast extract, lactulose, L-cystein hydrochloride and skim milk) for lactic acid bacteria were added to the whey. The pH of the solution is adjusted to a range from 6 to 7. The resulting solution is then subjected to heating to sterilize the solution. At the same time whey proteins (which are susceptibel to heat) denature, coagulate and precipitate. This resulting mixture is used as a culture medium for the lactic bacteria in the next step. In this way pre-treated whey is fermented in one fermentation step by means of lactid-acid-producing bacteria by heating and controlling of the pH-data. The precipitated proteins are separated by centrifugation and decomposed with a mineral acid or a proteolytic enzyme to produce a seasoning liquid A. The supernatant phase is neutralized and concentrated to produce a seasoning liquid B, and admixed with seasoning liquid A. The aimed product is a tasty seasoning from whey.

The procedure described in EP-A 0 147 517 ends also in a whey product which is used in the food industry. It describes a method of removing malodorous or unpleasant taste imparting substances from microbial cells or fermentation liquors, especially fermented whey by lactic acid bacteria.

The procedure according to EP-A 0 147 517 is characterized thereby that steam under pressure is blown into the fermented whey. Afterwards the whey is transferred in a vacuum atmosphere and is cooled down. The fermentation of the whey occurs with a lactic-acid-producing bacterium.
These documents do not describe a procedure how to obtain whey (which is the by-product of cheese manufacture) which may be pumped into environment without problems.

We thus find that by employing the procedure comprising the object of this patent, the advantage is significantly valuable in themselves, and is achieved by achieving the elimination of the contaminating effects of the whey produced as a by-product in cheese manufacture, as has been previously mentioned.

As regards of the aforesaid advantage, we are confronted with the need of eliminating solid substances existing in the whey, basically lactose, entailing percentages of 4.2% - 4.7% of the contents, in a medium having a high moisture content (93%-94%) and, thus, under conditions greatly favoring cases of undesirable, uncontrolled fermentation taking place, on the bacteria count being quite high. Special mention must also be made of the possible presence in this whey of highly contaminating substances, such as nitrites and some highly toxic metal salts.

The procedure for treating whey produced as a by-product of cheese manufacture to remove contaminating substances like proteins, including albumins and globulins, and lactose from the whey , comprise the following steps:
- installing a first inoculum fermenter (1), equipped with a steam-supply system (3), a temperature-control system and a pH meter;
- inseminating of an inoculum comprising *Lactobacillus casei* and lactose into said first fermenter (1) and fermentation so as to yield an inoculum;
- transferring the inoculum in a second, industrial fermenter (2) containing whey, whereby the fermenter (2) is connected to fermenter (1) and to an alkali tank (4),
- maintaining acidity of fermenter (2) at the pH range of 5-6 for the growth of said *Lactobacillus casei,* by means of a metering pump set up in said alkali tank (4) under control of a second pH-meter installed for regulating acidity,
- applying steam of up to 120 °C and 1,2 atmospheres of overpressure to the industrial fermenter (2), so that at the same time as the fermentation fluid is sterilized, the proteins in said whey precipitate therein,
- routing the proteins of the whey from an outlet of the fermenter (2) through a filter press (5) to separate them,
- and routing the fluid through an ion-exchange resin column (7), so as to remove the nitrites and the toxic metal substances.
It is preferred that said procedure is carried out using deproteinized or untreated whey at a temperature within the 30 ° - 40 °C range, preferably at 38 °C. The acidity of said second fermenter (2) is preferably maintained at a pH of 5,4.

In the studies and experiments conducted for the purpose of developing the procedure described in this account, it has been found that Lactobacillus Casei, which is of the homofermenting type, produces, as the result of the fermentation of lactose, an L-lactic acid in proportions of over 97.5%, depending on the rate at which the same is produced under variable experimental conditions, some of the most worthy of note being the concentration of the substrate, the temperature, the concentration of the inoculum and the pH of the system.

The procedure in question is essentially characterized by availing of an insemination fermenter in which an insemination of an inoculum comprising L. casei and lactose is fermented so as to yield an inoculum, said inoculum fermenter being equipped with a steam-supply system which affords the possibility of sterilizing the fermenter, which is additionally equipped with a temperature control system and a pH meter.

The inoculum thus prepared is placed in a second industrial fermenter which is connected to an alkali tank for the purpose of making it possible to maintain the acidity in this fermenter at the optimum levels for the microorganism to grow. For this purpose, a metering pump will be installed on said tank and will be enable by a pH meter employed for the purpose of controlling acidity. During this stage, the fermentation can be achieved using deproteinized whey or untreated whey, by varying the proportion of the lactic acid content within the range of 95%, in the first case of treated whey, and 65%-75%, in the second case. Steam up to 120°C and 1.2 atmospheres of overpressure must be supplied to the industrial fermenter. Thus, at the same time that the fermentation product is sterilized, the proteins in the whey, albumins and globulins precipitate and are routed to a filter press.

The production of D-lactic or L-lactic isomers, which is no subject-matter of the present invention, depends upon several factors as is inferred from the research conducted for the purpose of developing this process, it having been discovered that the control of the pH and the temperature at which this process is carried out leads to the inoculum inseminated outweighing the microorganisms remaining in the whey. Also of importance is the nature of the base used in the alkalinization of the system, as is revealed in the results shown in Table 1 in following.

**TABLE 1**

| Determination of L(+) and D(-) Lactic Acid Results given in g/l | | | | | |
|---|---|---|---|---|---|
| Sample | pH | Base | Whey(1) | L(+)lactic | D(-)lactic |
| 1 | 1.60 | NaOH | Combined | 18.8 | 0.35 |
| 2 | 1.86 | NaOH | Combined | 21.8 | 1.50 |
| 3 | 1.53 | NH₄OH | Combined | 10.9 | 7.70 |
| 4 | 1.49 | Ca(OH)₂ | Combined | 2.5 | 15.70 |

| | | | | | |
|---|---|---|---|---|---|
| (1) considered as being combined whey: (goat's milk + cow's milk) | | | | | |

L-lactic acid production falls within the 93%-96% range when NaOH is used, and totals up to 58% when NH₄OH is used, the results being the opposite in the case of using Ca(OH)₂, thus making the use thereof highly inadvisable.

The final stage of this process includes the subsequent routing of the fluid from the industrial fermenter through a filter press, a microfilter and some ion-exchange resin columns, these components being installed in this order at the outlet of the main fermenter.

In the aforesaid filter press, the whey proteins, albumins and globulins are separated from the system, proceeding directly in following to eliminating the bacterial cells by routing the liquid from said filter press through hollow-fiber microfilters.

Finally, by means of an ion-exchange system, a product having of pH below 2 and containing no nitrites or toxic metal substances is achieved. The toxicological analysis of the lactic acid thus obtained is shown in Table 2 as provided below.

**TABLE 2**

| L-LACTIC ACID TOXICOLOGICAL ANALYSIS | |
|---|---|
| Nitrites | None found |
| Chlorides | 2.5 mg/l |
| Phosphates | 3.8 mg/l |
| Calcium Salts | 2.5 mg/l |
| Magnesium Salts | 0.65 mg/l |
| Toxic Metals | None found |
| L-Lactic Acid | 30.88 g/l |
| D-Lactic Acid | 2.11 g/l |
| Lactose | 12.2 g/l |
| Proteins | 3.01 g/l |

It is of interest to point out that following the implementation of the procedure described in this patent, an excellent degree of efficiency is achieved in the production of lactic acid with a total lack of nitrites and of toxic metals, determining that the resulting product is comprised of a high-quality L-lactic acid which can be used as a food preservative.

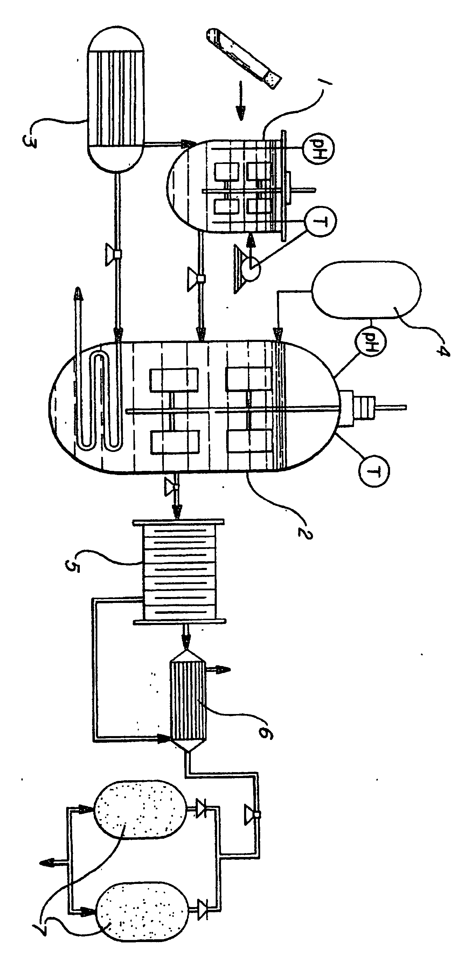

In order to provide for a more ready comprehension of the object of this patent, for illustrative purposes and without a restrictive scope, a description is furnished as to the details of one possible embodiment of the invention, making reference to the attached drawing, on which the diagrammed arrangement of the system designed for carrying out this procedure is shown.

As is shown in the aforesaid drawing, the system is comprised of an insemination fermenter (1), preferably of a capacity within the 5%-10% range of an industrial fermenter (2) for producing lactic acid, to which it is directly connected.

The fermenter (1) is assisted by an agitator system and a steam unit (3) which provides it with the necessary temperature for the operation thereof.

The industrial fermenter (2) is also equipped with a temperature-control system, is connected to an alkali tank (4) installed to control the pH on the interior of the fermenter. This fermenter (2) is equipped with the pertinent agitator, it having been established that the upper blades of the agitator be located at a depth, as regards the surface of the liquid, equivalent to three times the diameter of the agitator.

Following the procedure, it is shown on the drawing that, after the fluid has been processed in the fermenter (2), it is subsequently routed through the filter press (5) and the microfilters (6), to then finally be routed into an ion-exchange circuit (7).

As is shown in the Figure, this circuit is comprised of cylindrical receptacles (exchange columns) containing suitable exchange resins. This device affords the possibility of the alternating regeneration of the saturated resin-carrying columns.

## Claims

1. A procedure for treating whey produced as a by-product of cheese manufacture to remove contaminating substances like proteins, including albumins and globulins, and lactose from the whey , comprising the steps of
- installing a first inoculum fermenter (1), equipped with a steam-supply system (3), a temperature-control system and a pH meter;
- inseminating of an inoculum comprising *Lactobacillus casei* and lactose into said first fermenter (1) and fermentation so as to yield an inoculum;
- transferring the inoculum in a second, industrial fermenter (2) containing whey, whereby the fermenter (2) is connected to fermenter (1) and to an alkali tank (4),
- maintaining acidity of fermenter (2) at the pH range of 5-6 for the growth of said *Lactobacillus casei,* by means of a metering pump set up in said alkali tank (4) under control of a second pH-meter installed for regulating acidity,
- applying steam of up to 120 °C and 1,2 atmospheres of overpressure to the industrial fermenter (2), so that at the same time as the fermentation fluid is sterilized, the proteins in said whey precipitate therein,
- routing the proteins of the whey from an outlet of the fermenter (2) through a filter press (5) to separate them,
- and routing the fluid through an ion-exchange resin column (7), so as to remove the nitrites and the toxic metal substances.

2. Procedure according to claim 1, wherein said procedure is carried out using deproteinized or untreated whey at a fermentation temperature within the 30 ° - 40 °C range, preferably at 38 °C.

3. Procedure according to claim 1 wherein the acidity of said second fermenter (2) is maintained at a pH of 5,4.

4. Procedure, according to claim 1 wherein said metering pump delivers alkaline base to maintain the acidity of said second fermenter (2), wherein the prefered alkaline base is sodium hydroxide.

5. Procedure according to claim 1, further comprising the step of routing said fluid from the outlet of said industrial fermenter (2) consecutively through a filter press (5) and a microfilter (6).

6. Procedure according to claim 5 wherein the fluid having passed the filter press (5) is passed through a hollow-fiber microfilters (6) to eliminate bacterial cells.

## Patentansprüche

1. Verfahren zur Behandlung von Molke, die als Nebenprodukt bei der Käseherstellung anfällt, um verunreinigende Substanzen wie Proteine, darunter Albumine und Globuline, und Lactose von der Molke abzutrennen, umfassend die Schritte
- Aufstellen eines ersten Impffermenters (1), der ausgestattet ist mit einem Dampfversorgungssystem (3), einem Temperatursteuersystem und einem pH-Meßgerät;
- Einimpfen eines Inokulums, umfassend *Lactobacillus casei* und Lactose, in den ersten Fermenter (1) und Fermentierung, um so ein Inokulum zu ergeben;
- Überführen des Inokulums in einen zweiten, industriellen Fermenter (2), der die Molke enthält, wobei der Fermenter (2) mit dem Fermenter (1) und einem Alkalitank (4) verbunden ist;
- Aufrechterhalten der Acidität von Fermenter (2) im pH-Bereich 5-6 zur Vermehrung des *Lactobacillus casei*, wobei eine Dosierpumpe verwendet wird, die im Alkalitank (4) bereitgestellt ist und von einem zweiten pH-Meßgerät gesteuert wird, das zur Regulierung der Acidität angeschlossen ist;
- Einleiten von Dampf mit bis zu 120°C und 1,2 Atmosphären Überdruck in den industriellen Fermenter (2), so daß gleichzeitig mit der Sterilisierung der Fermentationsflüssigkeit die Proteine in der Molke ausfallen;
- Führen der Proteine der Molke aus einem Auslaß des Fermenters (2) durch eine Filterpresse (5) zur Abtrennung derselben; und
- Führen der Flüssigkeit durch eine Säule (7) mit Ionenaustauscherharz, um so Nitrite und Substanzen toxischer Metalle abzutrennen.

2. Verfahren nach Anspruch 1, wobei das Verfahren mit entproteinierter oder unbehandelter Molke bei einer Fermentationstemperatur im Bereich von 30°C bis 40°C und vorzugsweise bei 38°C durchgeführt wird.

3. Verfahren nach Anspruch 1, wobei die Acidität des zweiten Fermenters (2) bei einem pH von 5,4 gehalten wird.

4. Verfahren nach Anspruch 1, wobei mit der Dosierpumpe eine alkalische Base zugeführt wird, um die Acidität des zweiten Fermenters (2) aufrechtzuerhalten, wobei die bevorzugte alkalische Base Natriumhydroxid ist.

5. Verfahren nach Anspruch 1, des weiteren umfassend den Schritt, daß die Flüssigkeit aus dem Auslaß des industriellen Fermenters (2) nacheinander durch eine Filterpresse (5) und ein Mikrofilter (6) geführt wird.

6. Verfahren nach Anspruch 5, wobei die Flüssigkeit, die die Filterpresse (5) passiert hat, zur Beseitigung von Bakterienzellen durch ein Hohlfaser-Mikrofilter (6) geleitet wird.

## Revendications

1. Procédé de traitement du lactosérum produit en tant que sous-produit de la production de fromage, destiné à enlever du lactosérum les substances contaminantes telles que les protéines, y compris les albumines et les globulines, et le lactose, comprenant les étapes suivantes :
- mise en place d'un premier fermenteur à inoculum (1) doté d'un système d'alimentation en vapeur (3), d'un système de régulation de température et d'un pH-mètre,
- ensemencement d'un inoculum comprenant de *Lactobacillus casei* et du lactose dans ledit premier fermenteur (1) et fermentation de façon à produire un inoculum,
- transfert de l'inoculum dans un second fermenteur industriel (2) contenant le lactosérum, le fermenteur (2) étant raccordé au fermenteur (1) et à une cuve de produit alcalin (4),
- maintien de l'acidité du fermenteur (2) dans une plage de pH de 5 à 6 pour la croissance de dit *Lactobacillus casei*, au moyen d'une pompe doseuse installée dans ladite cuve de produit alcalin (4) sous le contrôle d'un second pH-mètre destiné à réguler l'acidité,
- alimentation avec de la vapeur jusqu'à une température de 120° C et de 1,2 atmosphères de pression relative vers le fermenteur industriel (2), de façon à stériliser le fluide de fermentation tout en faisant précipiter en même temps les protéines contenues dans le dit lactosérum,
- acheminement des protéines du lactosérum à partir d'une sortie du fermenteur (2) à travers un filtre-presse (5) pour les séparer,
- et acheminement du fluide à travers une colonne de résines à échange d'ions (7), de façon à éliminer les nitrites et les substances de métal toxiques.

2. Procédé selon la revendication 1, dans lequel ledit procédé est réalisé en utilisant du lactosérum déprotéiné ou non traité à une température de fermentation comprise entre 30 et 40° C, de préférence de 38° C.

3. Procédé selon la revendication 1, dans lequel on maintient l'acidité dudit second fermenteur (2) à un pH de 5,4.

4. Procédé selon la revendication 1, dans lequel la dite pompe doseuse distribue une base alcaline pour maintenir l'acidité dudit second fermenteur (2), la base alcaline étant de préférence de l'hydroxyde de sodium.

5. Procédé selon la revendication 1, comportant en outre une étape d'acheminement dudit fluide à partir de la sortie dudit fermenteur industriel (2) à travers un filtre-presse (5), puis un microfiltre (6).

6. Procédé selon la revendication 5, dans lequel le fluide, après avoir passé au travers du filtre-presse (5), passe à travers un microfiltre à fibres creuses (6) afin d'éliminer les cellules bactériennes.
